(19) **Europäisches Patentamt / European Patent Office / Office européen des brevets**

(11) **EP 4 282 984 A1**

(12) **EUROPEAN PATENT APPLICATION**
published in accordance with Art. 153(4) EPC

(43) Date of publication:
**29.11.2023 Bulletin 2023/48**

(21) Application number: **22862405.2**

(22) Date of filing: **17.08.2022**

(51) International Patent Classification (IPC):
**C12Q 1/6886** (2018.01)    **G16B 40/00** (2019.01)

(52) Cooperative Patent Classification (CPC):
**C12Q 1/6886; G16B 40/00**

(86) International application number:
**PCT/KR2022/012252**

(87) International publication number:
**WO 2023/191197 (05.10.2023 Gazette 2023/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.03.2022 KR 20220038857**

(71) Applicant: **IMB DX, Inc.**
**Geumcheon-gu**
**Seoul 08506 (KR)**

(72) Inventors:
• **JEONG, Seong Mun**
  **Pyeongtaek-si, Gyeonggi-do 18008 (KR)**
• **LEE, Wook Jae**
  **Seoul 06310 (KR)**
• **KIM, Su Yeon**
  **Daejeon 34191 (KR)**
• **KIM, Hwang Phill**
  **Yongin-si, Gyeonggi-do 16990 (KR)**
• **MOON, Sung Tae**
  **Seoul 05224 (KR)**
• **KIM, Tae You**
  **Seoul 03009 (KR)**

(74) Representative: **Cabinet Nony**
**11 rue Saint-Georges**
**75009 Paris (FR)**

(54) **METHOD FOR CONSTRUCTION OF MULTI-FEATURE PREDICTION MODEL FOR CANCER DIAGNOSIS**

(57) The present invention relates to a method for preparing a multi-analytical prediction model for cancer diagnosis and a method of providing information for cancer diagnosis using the same. The method of preparing a multi-analytical prediction model for cancer diagnosis according to one embodiment of the present invention and the method of providing information for cancer diagnosis using the prediction model have advantages in that it is possible to diagnose cancer with high accuracy and sensitivity and to diagnose cancer at an early stage.

Fig.6

EP 4 282 984 A1

**Description**

**Technical Field**

The present invention relates to a method for preparing a multi-analytical prediction model for cancer diagnosis and a method of providing information for cancer diagnosis using the same.

**Background Art**

In recent years, cell-free DNA (cfDNA) or circulating tumor DNA (ctDNA) present in blood has been used to detect cancer. In healthy persons, most of the DNA is released from hematopoietic cells, but in cancer patients, cfDNA contains ctDNA released from dying tumor cells into the blood. This ctDNA contains genetic mutations related to cancer, and monitoring of these genetic mutations enables early detection of cancer before the occurrence of lesions, analysis of responses to specific cancer treatments, discovery of mechanisms for generating resistance to anticancer drugs, detection of the presence of residual cancer, and the like.

Meanwhile, whole-genome DNA methylation mapping leverages a number of epigenetic alterations that may be used to distinguish ctDNA from normal circulating cell-free DNA. For example, some tumor types, such as ependymomas, can have extensive DNA methylation aberrations without any significant recurrent somatic mutations.

In recent years, various cancer diagnosis techniques such as CancerSEEK, PanSeer, and GRAIL MCED test have been developed using cfDNA. Since these techniques mainly use target sequencing to perform diagnosis using only methylation patterns, protein level, or mutation in specific regions, they have a limitation in that only a limited number of markers are used. Thus, there is a need for a predictive model for cancer diagnosis with high sensitivity and accuracy.

Accordingly, the present invention is intended to propose an analytical prediction model for cancer diagnosis prepared through machine learning using the data alone or ensemble that are extracted by applying various features such as methylation pattern fraction, copy number ratio, and fragment size ratio.

**DISCLOSURE**

**Technical Problem**

An object of one aspect of the present invention is to provide a method for preparing a multi-analytical prediction model for cancer diagnosis, the method comprising steps of: a) selecting segments necessary for cancer diagnosis prediction from CpG site information for a human reference genome; b) obtaining whole-genome methylation sequencing information for cfDNA from two or more liquid biopsy samples; c) applying a methylation pattern fraction feature, among the obtained whole-genome methylation sequencing information for cfDNA, to the selected segments, and additionally applying, to the selected segments, at least one feature selected from the group consisting of a copy number ratio and a fragment size ratio, thereby extracting feature data; and d) generating a cancer diagnosis prediction model through machine learning using at least one of the extracted feature data.

An object of another aspect of the present invention is to provide a method for providing information for cancer diagnosis, the method comprising steps of: a) obtaining whole-genome methylation sequencing information for cfDNA from a liquid biopsy sample of a subject patient; and b) detecting the presence or absence of cancer and/or cancer-derived tissue by applying the whole-genome methylation sequencing information for cfDNA to a multi-analytical prediction model for cancer diagnosis.

**Technical Solution**

One embodiment of the present invention provides a method for preparing a multi-analytical prediction model for cancer diagnosis, the method comprising steps of: a) selecting segments necessary for cancer diagnosis prediction from CpG site information for a human reference genome; b) obtaining whole-genome methylation sequencing information for cfDNA from two or more liquid biopsy samples; c) applying a methylation pattern fraction feature, among the whole-genome methylation sequencing information for cfDNA obtained in step b), to the segments selected in step a), and additionally applying, to the segments, at least one feature selected from the group consisting of a copy number ratio and a fragment size ratio, thereby extracting feature data; and d) generating a cancer diagnosis prediction model through machine learning using at least one of the feature data extracted in step c).

In one embodiment of the present invention, step a) may comprise selecting a segment, which satisfies the following conditions, as a segment necessary for cancer diagnosis prediction:

    1) the segment comprises CpG sites whose sequencing depth in healthy persons is 3 or more;

2) the distance between CpG sites is less than 100 bp, and the segment comprises at least 3 CpG sites;

3) the segment is divided when the segment length exceeds 1 kb;

4) sex chromosomes are excluded; and

5) the average sequencing depth of the segments in 900 or more, excluding lower 10% in healthy persons, exceeds 3.

In one embodiment of the present invention, the liquid biopsy sample may be blood from a healthy person or a cancer patient.

In one embodiment of the present invention, the methylation pattern fraction may be determined by calculating the ratio of the number of methylated Cs among CpGs in all reads for the segments selected in step a).

In one embodiment of the present invention, the methylation pattern fraction may be determined by calculating the ratio of methylated CpGs that are opposite to the predefined methylation pattern of healthy persons for the segments selected in step a).

In one embodiment of the present invention, the copy number ratio may be determined by dividing the entire genome into bins, calculating the depth value for each bin, dividing the depth value for each bin of the subject's sample by a reference value which is the median value of the depth for each bin from whole-genome methylation sequencing information for cfDNA of healthy persons, and then calculating a log value.

In one embodiment of the present invention, the fragment size ratio may be determined by classifying fragments, mapped to each of the segments selected in step a), into first fragments of 100 bp to 150 bp and second fragments of 150 bp to 220 bp, and calculating the number of the first segments and the number of the second segments as a log ratio.

In one embodiment of the present invention, the cancer diagnosis prediction model may detect the presence or absence of cancer and/or cancer-derived tissue.

Another aspect of the present invention provides a method for providing information for cancer diagnosis, the method comprising steps of: a) obtaining whole-genome methylation sequencing information for cfDNA from a liquid biopsy sample of a subject patient; and b) detecting the presence or absence of cancer and/or cancer-derived tissue by applying the whole-genome methylation sequencing information for cfDNA obtained in step a) to the multi-analytical prediction model for cancer diagnosis prepared through the method of claim 1.

## Advantageous Effects

The method of preparing a multi-analytical prediction model for cancer diagnosis according to one embodiment of the present invention and the method of providing information for cancer diagnosis using the prediction model have advantages in that it is possible to diagnose cancer with high accuracy and sensitivity and to diagnose cancer at an early stage.

## Brief Description of Drawings

FIG. 1 shows an example of a process of selecting segments necessary for cancer diagnosis prediction using CpG information for a human reference genome according to one embodiment of the present invention.

FIG. 2 shows an example of a method of extracting data for an average methylation fraction according to one embodiment of the present invention.

FIG. 3 shows an example of a method of extracting data for an abnormal methylation pattern fraction according to one embodiment of the present invention.

FIG. 4 shows an example of a method of extracting data for a copy number ratio according to one embodiment of the present invention.

FIG. 5 depicts graphs showing the difference in fragment size distribution between cfDNA of healthy persons and cfDNA of colorectal cancer patients.

FIG. 6 is a schematic diagram showing a process of generating a predictive model for cancer diagnosis by machine learning for data extracted according to one embodiment of the present invention.

FIG. 7 depicts data showing the results of predicting the presence or absence of cancer based on each feature using a cancer prediction model (IsCancer) according to one embodiment of the present invention.

FIG. 8 depicts data showing the results of predicting the presence or absence of cancer based on an ensemble of four features using a cancer prediction model (IsCancer) according to one embodiment of the present invention.

FIG. 9 depicts data showing the results of predicting cancer-derived tissue based on each feature using a cancer prediction model (Tissue-of-Origin) according to one embodiment of the present invention.

FIG. 10 depicts data showing the results of predicting cancer-derived tissue based on an ensemble of four features using a cancer prediction model (Tissue-of-Origin) according to one embodiment of the present invention.

**Best Mode**

One aspect of the present invention provides a method for preparing a multi-analytical prediction model for cancer diagnosis, the method comprising steps of: a) selecting segments necessary for cancer diagnosis prediction from CpG site information for a human reference genome; b) obtaining whole-genome methylation sequencing information for cfDNA from two or more liquid biopsy samples; c) applying a methylation pattern fraction feature, among the whole-genome methylation sequencing information for cfDNA obtained in step b), to the segments selected in step a), and additionally applying, to the segments, at least one feature selected from the group consisting of a copy number ratio and a fragment size ratio, thereby extracting feature data; and d) generating a cancer diagnosis prediction model through machine learning using at least one of the feature data extracted in step c).

It is known that, in the blood of cancer patients, circulating tumor DNA (ctDNA) and cell-free DNA (cfDNA) from primary cancer circulate together. In particular, it is known that the amount of the DNA is larger in cancer patients than in healthy persons and differs between before and after chemotherapy, and when cancer recurs after treatment, the amount of ctDNA increases. During studies on cancer diagnosis technology using cfDNA, the present inventors have made extensive efforts to overcome the limitations of a diagnosis method that uses the methylation pattern of a specific region based on existing targeted sequencing, and as a result, have prepared an analytical prediction model for cancer diagnosis with high sensitivity and accuracy through machine learning using data extracted by applying various features such as methylation pattern fraction, copy number ratio, and fragment size ratio, and have verified that it is possible to effectively diagnose cancer diagnosis through the analytical prediction model, thereby completing the present invention.

Hereinafter, the method for preparing a multi-analytical prediction model for cancer diagnosis according to the present invention will be described in detail.

First, in the method of the present invention, step a) of selecting segments necessary for cancer diagnosis prediction from CpG site information for a human reference genome is performed.

In the genomic DNA of mammalian cells, a fifth nucleotide called 5-methylcytosine (5-mC), in which a methyl group is attached at the 5-carbon of the cytosine ring, exists in addition to A, C, G and T. Methylation of 5-methylcytosine occurs only at the C of the CG dinucleotide (5'-CG-3'), called the CpG site, and 5-mC at the CpG site is spontaneously deaminated to thymine (T). Thus, the CpG site frequently undergoes most epigenetic alterations in mammalian cells. The CpG site may be present in a promoter region, intron region, exon region or the like of a gene included in the genome.

According to one embodiment of the present invention, step a) may comprise selecting a segment, which satisfies the following conditions, as a segment necessary for cancer diagnosis prediction:

> 1) the segment comprises CpG sites whose sequencing depth in healthy persons is 3 or more;
> 2) the distance between CpG sites is less than 100 bp, and the segment comprises at least 3 CpG sites;
> 3) the segment is divided when the segment length exceeds 1 kb;
> 4) sex chromosomes are excluded; and
> 5) the average sequencing depth of the segments in 900 or more, excluding lower 10% in healthy persons, exceeds 3.

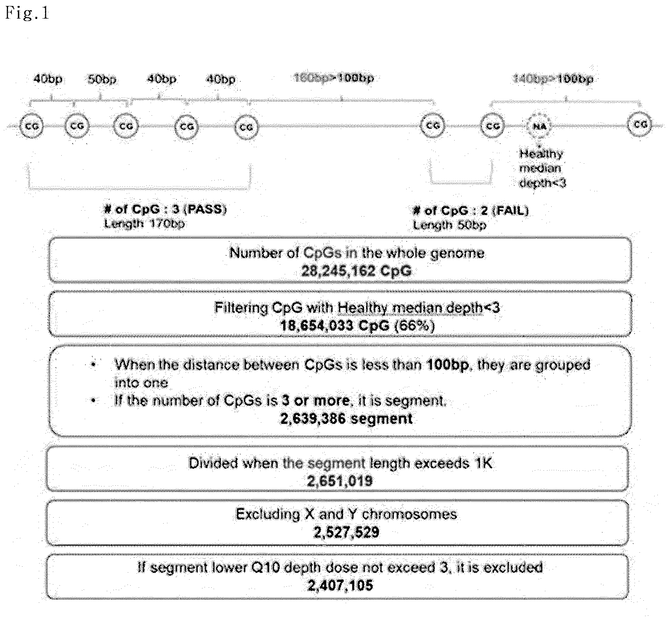

FIG. 1 shows an example of a process of selecting segments necessary for cancer diagnosis prediction using CpG information for a human reference genome according to one embodiment of the present invention. In this example, CpG information was obtained from the GRCh37 version of the human reference genome sequence downloaded from the UCSC Genome Browser. Referring to FIG. 1 showing the process of selecting segments necessary for cancer diagnosis prediction, the total number of CpG sites in the human genome is 28,245,162, and the number of CpG sites whose sequencing depth in healthy persons is 3 or more is about 18,654,033 (about 66%). Among them, there are 2,639,386 segments where the distance between CpG sites is less than 100 bp and the number of CpG sites is 3 or more. Of these, 2,651,019 segments are selected by dividing a segment exceeding 1 kb. Then, 2,527,529 segments are selected by excluding sex chromosome segments, and finally, 2,407,105 segments are selected by selecting segments whose sequencing depth in the lower 100 of healthy persons exceeds 3.

Then, in the method of the present invention, step b) of obtaining whole-genome methylation sequencing information for cfDNA from two or more liquid biopsy samples is performed.

According to one embodiment of the present invention, the liquid biopsy sample may include a liquid sample from a healthy person or a cancer patient, such as whole blood, serum, plasma, saliva, sputum, cerebrospinal fluid, or urine. Most preferably, the liquid biopsy sample is blood.

In the present invention, "cell-free DNA" or "cfDNA" refers to a fragment of a nucleic acid found outside of a cell (e.g., bodily fluid), wherein the bodily fluid includes blood, cerebrospinal fluid, saliva, or urine, without being limited thereto. The cfDNA may be derived from a subject (e.g., from a cell of the subject) or from a source other than the subject (e.g., from a viral infection).

Extraction of cfDNA may be performed according to a method known in the art, and methylation of the extracted cfDNA may be confirmed, for example, by preparing a DNA library through a methylation method known in the art, and then obtaining whole-genome methylation sequencing information through next-generation sequencing (NGS). Next-generation sequencing techniques are described in detail in Metzker, M. (2010) Nature Biotechnology Reviews 11:31-46, which is incorporated herein by reference.

In the present invention, "methylation" means that a methyl group is attached to a base of DNA. Preferably, methylation in the present invention means methylation that occurs at cytosine of the CpG sites in the human genome. In general, when methylation occurs, it hinders transcription factor binding, thereby inhibiting the expression of a specific gene, and conversely, when unmethylation or hypomethylation occurs, expression of a specific gene increases.

Next, in the present invention, step c) of applying a methylation pattern fraction feature, among the whole-genome methylation sequencing information for cfDNA obtained in step b), to the segments selected in step a), and additionally applying, to the segments, at least one feature selected from the group consisting of a copy number ratio and a fragment size ratio, thereby extracting feature data, is performed.

According to one embodiment of the present invention, the methylation pattern fraction may be determined by calculating the ratio of the number of methylated Cs among CpGs in all reads for the segments selected in step a). In the present specification, the methylation pattern fraction determined as described above is defined as "average methylation fraction (AMF)".

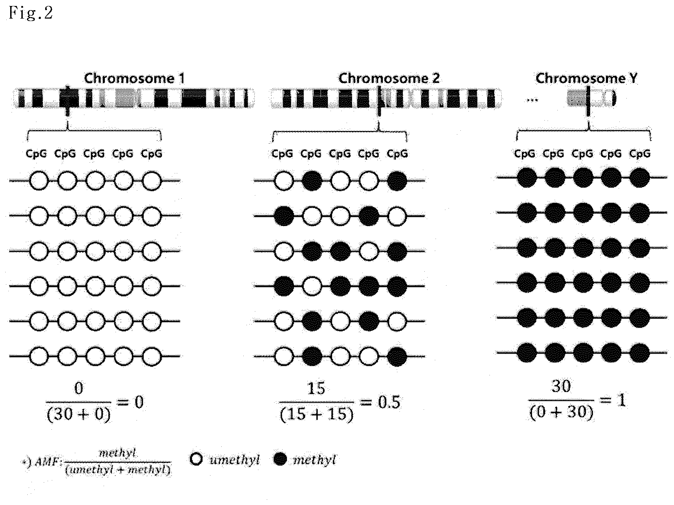

FIG. 2 shows an example of a method of extracting data for an average methylation fraction. For example, assuming that there are 24 CpG sites in all reads, the ratio may be calculated according to the number of methylated Cs among the CpG sites. In this case, as shown in FIG. 2, the number of methylated Cs is calculated only for cytosine included in the segments, and the average methylation fraction value may be extracted according to Equation I below. The average methylation fraction value extracted by this method is between 0 and 1.

[Equation I]

$$AMF_i = \frac{\sum_{j \in C_i} M_j}{\sum_{j \in C_i}(M_j + U_j)}$$

wherein Ci denotes i-th segment obtained in step 1), $M_j$ denotes the number of methylated Cs at j-th CpG in Ci, and $U_i$ denotes the number of unmethylated Cs at j-th CpG in Ci.

According to one embodiment of the present invention, the methylation pattern fraction may be determined by calculating the ratio of methylated CpGs that are opposite to the predefined methylation pattern of healthy persons for the segments selected in step a). In the present specification, the methylation pattern fraction determined as described above is defined as "abnormal methylation pattern fraction (AMPF)".

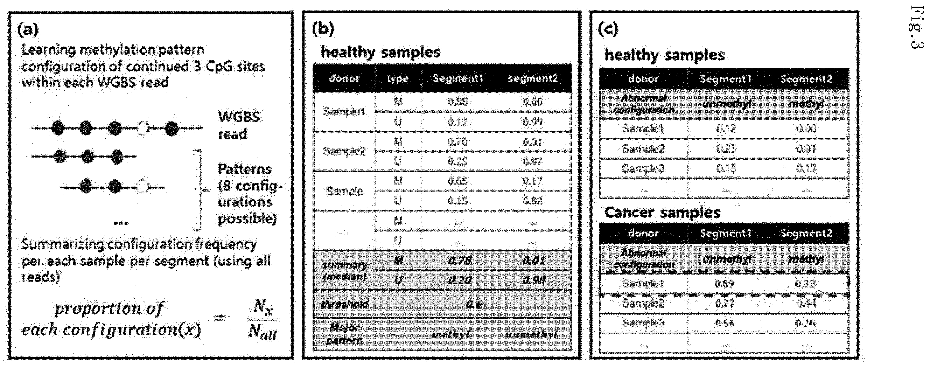

FIG. 3 shows an example of a method of extracting data for an abnormal methylation pattern fraction. As shown in FIGS. 3(a) to 3(c), first, a methylation pattern within each whole-genome methylation sequencing (WGMS) read is configured, configuration frequency per each sample is extracted, and then the methylation pattern of healthy persons is defined for each segment. Then, the level of the methylation pattern opposite to that of healthy persons is quantified, and a value is extracted by calculating the proportion of an abnormal methylation pattern. For example, if the major pattern of segment 1 for healthy samples is methylation and the methylation level of sample 1 of a cancer patient is 0.11, the proportion of abnormal methylation pattern of segment 1 in sample 1 is 0.89 (box mark in FIG. 3(c)).

According to one embodiment of the present invention, the copy number ratio may be determined by dividing the entire genome into bins, calculating the depth value for each bin, dividing the depth value for each bin of the subject's sample by a reference value which is the median value of the depth for each bin from whole-genome methylation sequencing information for cfDNA of healthy persons, and then calculating a log value.

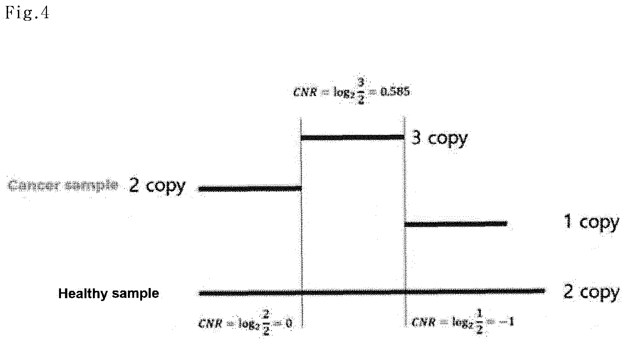

FIG. 4 shows an example of a method of extracting data for a copy number ratio. It is very difficult to quantify cfDNA copy number variation, but it is possible to collect information on copy number variation for each sample from whole-genome data. First, the entire genome is divided into bins (e.g., in units of 10 kb), and then the depth for each bin is calculated. Thereafter, the median value of the depth for each bin in the healthy sample is calculated and used as a reference value. The copy number ratio may be calculated by dividing the depth value for each bin of the sample of interest by the reference depth value calculated from the healthy sample and then taking the logarithm. As shown in the example of FIG. 4, if the median value of the depth for each bin of the healthy sample is 2 copies and the depth value for each bin of the subject's sample is 2 copies, the copy number ratio value becomes 0.

According to one embodiment of the present invention, the fragment size ratio may be determined by classifying fragments, mapped to each of the segments selected in step a), into first fragments of 100 bp to 150 bp and second fragments of 150 bp to 220 bp, and calculating the number of the first segments and the number of the second segments as a log ratio.

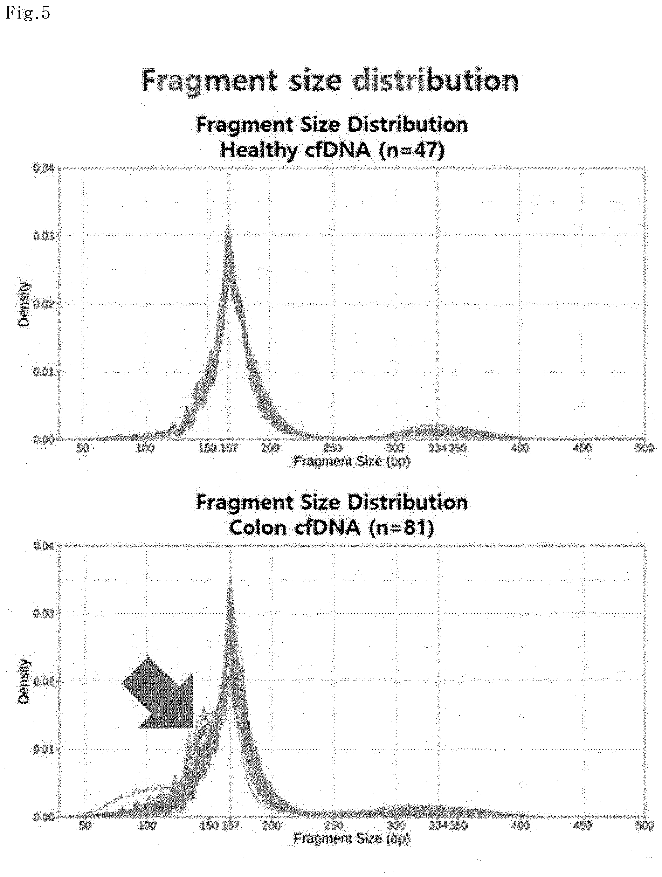

cfDNA circulating in the blood has molecular characteristics related to the size of DNA fragments. In particular, since cfDNA does not require a DNA fragmentation step in the NGS process, the size distribution of DNA fragments can be confirmed using only the cfDNA sequencing results. In addition, it has been reported that the fragment size is shortened by reflecting the patient's disease (e.g., cancer) or condition, and thus the fragment size may be used in a cancer diagnosis prediction model. FIG. 5 depicts graphs showing the difference in fragment size distribution between cfDNA of healthy persons and cfDNA of colorectal cancer patients. As shown in FIG. 5, it can be confirmed that the distribution of short fragments in the cfDNA fragment size of colorectal cancer patients is higher than that of healthy persons.

Extraction of data for the fragment size ratio can be performed as follows. For example, if the total number of fragments for the selected segment is 30, the number of the first fragments among fragments mapped to each segment is 10, and the number of the second fragments among fragments mapped to each segment is 20, the data value for the fragment size ratio may be -1 by the following calculation.

$$FragRatio = \log_2 \frac{10}{20} = -1$$

Meanwhile, extraction of data for the copy number ratio and fragment size ratio may be performed by binning the entire human genome.

Finally, in the method of the present invention, step d) of generating a cancer diagnosis prediction model through machine learning using the data extracted in step c) is performed.

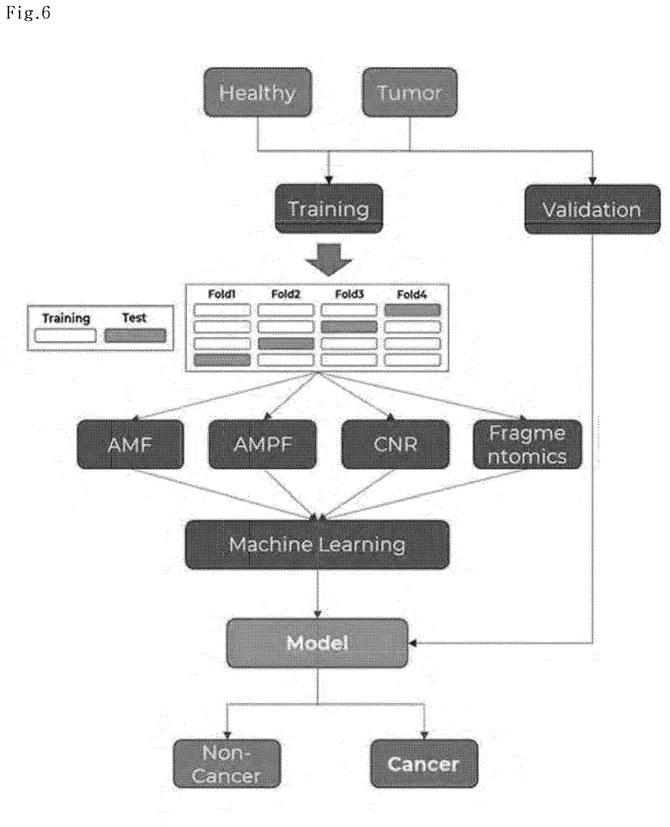

FIG. 6 shows a process of generating a cancer diagnosis prediction model through machine learning using data extracted by the method. Healthy person samples and cancer patient samples are divided into a training set and a validation set. The training set is subjected to 4-fold cross-validation to predict the evaluation of the final model before pre-validation, thereby generating a machine learning model. Models for respective features (methylation pattern fraction (AMF, AMPF), copy number ratio (CNR), and fragment size ratio (Fragmentomics)) may be constructed using classification models alone, such as support vector machine, random forest, and glmnet, or using an ensemble of several models. In addition, two ensemble models may be prepared using one or more features. According to one embodiment of the present invention, the cancer diagnosis prediction model can detect the presence or absence of cancer (IsCancer) and/or cancer-derived tissue (Tissue-of-Origin). In this case, the IsCancer ensemble model may be prepared using both healthy person and cancer patient samples, and the Tissue-of-Origin model may be prepared using cancer patient samples excluding healthy person samples. In addition, for validation evaluation, the Tissue-of-Origin model may be applied only to patients determined to have cancer in the IsCancer model, and performance may be evaluated using the training set and an independent validation set.

Another aspect of the present invention provides a method for providing information for cancer diagnosis, the method comprising steps of: a) obtaining whole-genome methylation sequencing information for cfDNA from a liquid biopsy sample of a subject patient; and b) detecting the presence or absence of cancer and/or cancer-derived tissue by applying the whole-genome methylation sequencing information for cfDNA obtained in step a) to the multi-analytical prediction model for cancer diagnosis prepared through the above-described method.

The method for providing information for cancer diagnosis according to the present invention detects the presence of cancer and/or cancer-derived tissue by applying whole-genome methylation sequencing information for cfDNA derived from a subject patient to the above-described multi-analytical prediction model for cancer diagnosis, and the analysis criterion and the validation method have been described above, and thus the description thereof will be omitted to avoid excessive complexity of the specification.

**Mode for Invention**

Hereinafter, one or more embodiments will be described in more detail with reference to examples. However, these examples are for explaining one or more embodiments in detail, and the scope of the present invention is not limited to these examples.

**Example 1. Whole-genome methylation sequencing method**

Plasma and peripheral blood mononuclear cells (PBMCs) were separated from the blood of subject patients, and cfDNA was extracted from the plasma using a cfDNA extraction kit (Promega, USA). The quality of the extracted cfDNA was confirmed using a TapeStation System (Agilent, USA). On 1 ng to 20 ng of the cfDNA whose quality was confirmed, a NGS DNA library preparation process for whole-genome methylation sequencing was performed. The DNA library was prepared through the processes of end repair, adapter ligation, methyl oxidation, DNA denaturation, cytosine deamination, and PCR amplification, and the library preparation process was performed using an enzymatic methyl-seq kit (New England Biolabs, USA). The quality of the prepared DNA library was confirmed using a TapeStation System (Agilent, USA). Then, for the produced DNA library, samples were mixed together according to the desired amount of NGS data (for example, to produce data of 100G sample A, 100G sample B, and 50G sample C, samples were mixed at a ratio of A:B:C = 2:2:1), and for the quality of NGS data, an appropriate amount of PhiX control library (Illumina, USA) was mixed. NGS was performed using Illumina's Novaseq system.

## Example 2. Results of prediction of presence or absence of cancer using cancer diagnosis prediction model (IsCancer)

Three types of cancer samples and healthy person samples were divided into training sets and validation sets in consideration of age and cancer stage information, and the presence or absence of cancer was predicted for each feature using the IsCancer model prepared according to the method of the present invention. Table 1 below shows the number of training sets and independent validation sets.

[Table 1]

| cfDNA | Healthy | Colorectal cancer (CRC) | Hepatocellular carcinoma (HCC) | Breast cancer (BC) |
|---|---|---|---|---|
| Training sets | 47 | 81 | 46 | 60 |
| Validation sets (independent) | 42 | 53 | 24 | 28 |

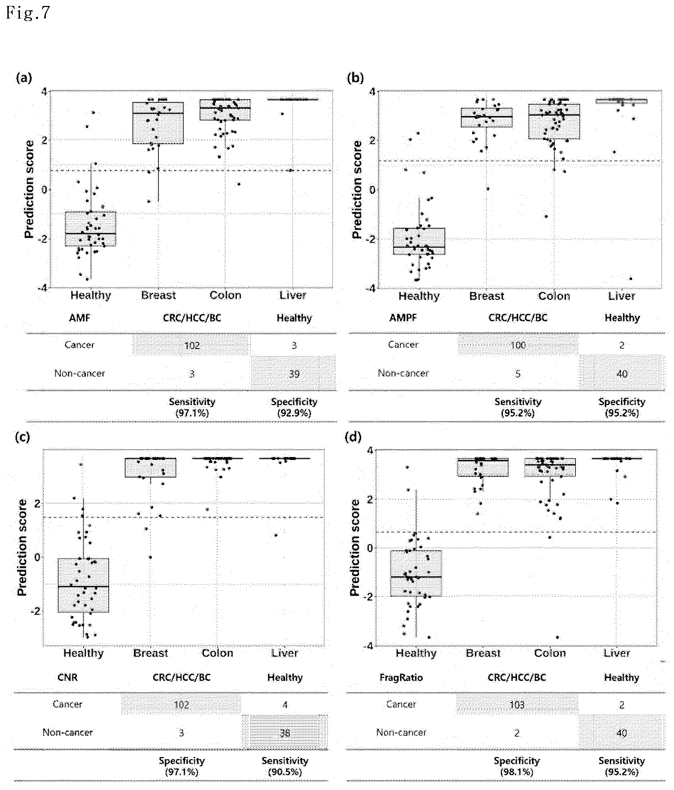

As a result of predicting the three types of cancer according to features, including methylation pattern fraction (AMF, AMPF) (FIGS. 7(a) and (b)), copy number ratio (CNR) (FIGS. 7(c)) and fragment size ratio (FragRatio) (FIGS. 7(d)), it was confirmed that, compared to healthy person samples, cancer and non-cancer were clearly distinguished from each other, specificities were 97.1% for AMF, 95.2% for AMPF, 97.1% for CNR, and 98.1% for FragRatio, and sensitivities were 92.9% for AMF, 95.2% for AMPF, 90.5% for CNR, and 92.9% for FragRatio, indicating that the presence or absence of cancer could be determined with high specificity and sensitivity.

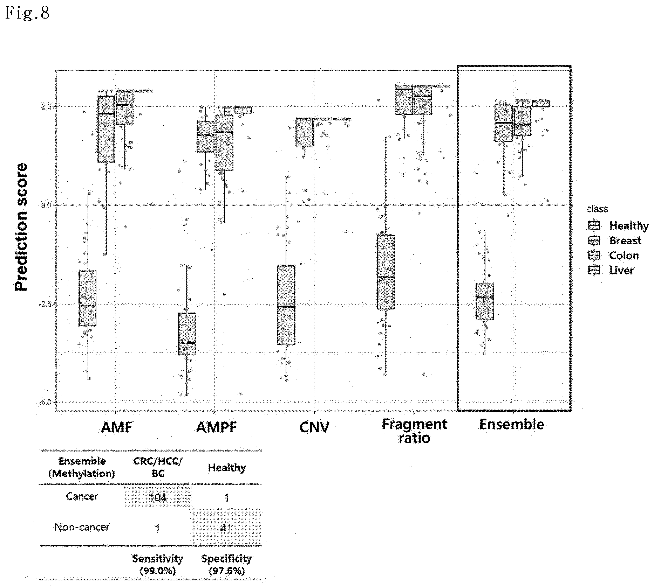

In addition, as a result of predicting the presence or absence of cancer using a prepared ensemble model for the above four features, it was confirmed that the variability of the score was stabilized compared to the result predicted according to each feature, and that the sensitivity increased to 99.0%, and the specificity increased to 97.6% (FIG. 8).

## Example 3. Results of prediction of cancer-derived tissue using cancer diagnosis prediction model (Tissue-of-Origin)

Three types of cancer samples were divided into training sets and validation sets in consideration of age and cancer stage information, and cancer-derived tissues for each feature were predicted using the Tissue-of-Origin model prepared according to the method of the present invention. Table 2 below shows the number of training sets and the number of independent validation sets.

[Table 2]

| cfDNA | Colorectal cancer (CRC) | Hepatocellular carcinoma (HCC) | Breast cancer (BC) |
|---|---|---|---|
| Training sets | 81 | 46 | 60 |
| Validation sets (independent) | 53 | 24 | 28 |

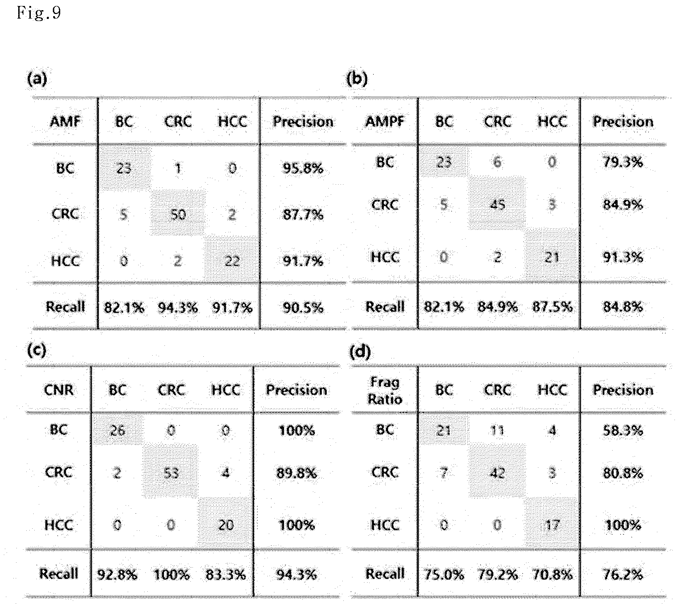

As a result of predicting three types of cancer-derived tissues according to features, including methylation pattern fraction (AMF, AMPF) (FIGS. 9(a) and 9(b)), copy number ratio (CNR) (FIG. 9(c)) and fragment size ratio (FragRatio) (FIG. 9(d)), it was confirmed that cancer-derived tissues could be predicted with high accuracy.

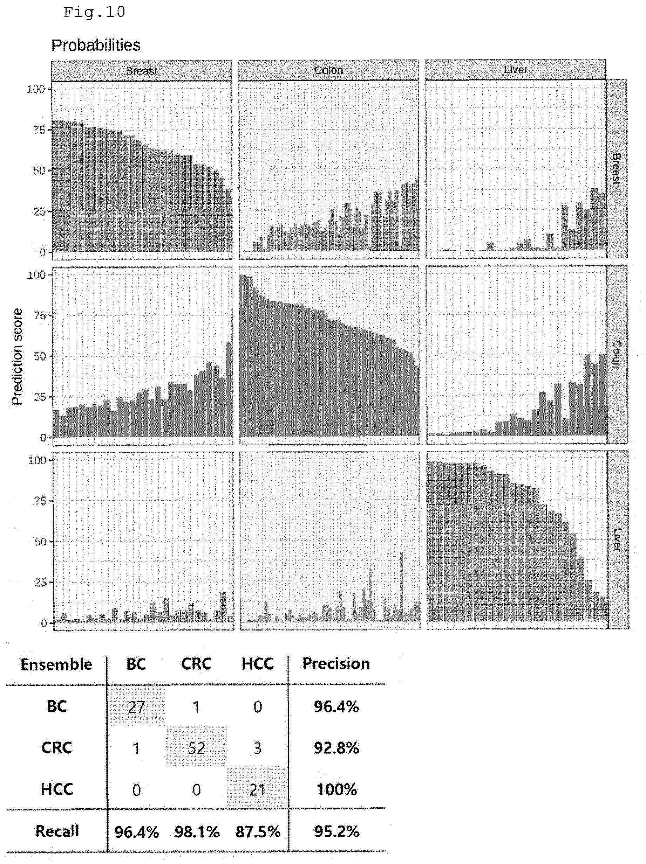

In addition, as a result of predicting cancer-derived tissues using a prepared ensemble model for the above-described four features, it could be confirmed that, compared to the results predicted according to each feature, the accuracy for each cancer type increased to 98.1%, and the accuracy for all of the cancers also increased to 95.2% (FIG. 10).

So far, the present invention has been described with reference to the preferred embodiments. Those skilled in the art will appreciate that the present invention can be implemented in modified forms without departing from the essential features of the present invention. Therefore, the disclosed embodiments should be considered in descriptive sense only and not for purposes of limitation. Therefore, the scope of the present invention is defined not by the detailed description of the present invention but by the appended claims, and all modifications within a range equivalent to the scope of the appended claims should be construed as being included in the present invention.

## Claims

1. A method for preparing a multi-analytical prediction model for cancer diagnosis, the method comprising steps of:

   a) selecting segments necessary for cancer diagnosis prediction from CpG site information for a human reference genome;
   b) obtaining whole-genome methylation sequencing information for cfDNA from two or more liquid biopsy samples;
   c) applying a methylation pattern fraction feature, among the whole-genome methylation sequencing information for cfDNA obtained in step b), to the segments selected in step a), and additionally applying, to the segments, at least one feature selected from the group consisting of a copy number ratio and a fragment size ratio, thereby extracting feature data; and
   d) generating a cancer diagnosis prediction model through machine learning using at least one of the feature data extracted in step c).

2. The method according to claim 1, wherein step a) comprises selecting a segment, which satisfies the following conditions, as a segment necessary for cancer diagnosis prediction:

   1) the segment comprises CpG sites whose sequencing depth in healthy persons is 3 or more;
   2) the distance between CpG sites is less than 100 bp, and the segment comprises at least 3 CpG sites;
   3) the segment is divided when the segment length exceeds 1 kb;
   4) sex chromosome segments are excluded; and
   5) the average sequencing depth of the segments in 900 or more, excluding lower 10% in healthy persons, exceeds 3.

3. The method according to claim 1, wherein the liquid biopsy sample is blood from a healthy person or a cancer patient.

4. The method according to claim 1, wherein the methylation pattern fraction is determined by calculating the ratio of the number of methylated Cs among CpGs in all reads for the segments selected in step a).

5. The method according to claim 1, wherein the methylation pattern fraction is determined by calculating the ratio of methylated CpGs that are opposite to the predefined methylation pattern of healthy persons for the segments selected in step a).

6. The method according to claim 1, wherein the copy number ratio is determined by dividing the entire genome into bins, calculating a depth value for each bin, dividing the depth value for each bin of the subject sample by a reference value which is the median value of the depth for each bin from whole-genome methylation sequencing information for cfDNA of healthy persons, and then calculating a log value.

7. The method according to claim 1, wherein the fragment size ratio is determined by classifying fragments, mapped to each of the segments selected in step a), into first fragments of 100 bp to 150 bp and second fragments of 150 bp to 220 bp, and calculating the number of the first segments and the number of the second segments as a log ratio.

8. The method according to claim 1, wherein the cancer diagnosis prediction model detects the presence or absence of cancer and/or cancer-derived tissue.

9. A method for providing information for cancer diagnosis, the method comprising steps of:

   a) obtaining whole-genome methylation sequencing information for cfDNA from a liquid biopsy sample of a subject patient; and
   b) detecting the presence or absence of cancer and/or cancer-derived tissue by applying the whole-genome methylation sequencing information for cfDNA obtained in step a) to the multi-analytical prediction model for cancer diagnosis prepared through the method of claim 1.

Fig.1

Fig.2

$$\frac{0}{(30+0)} = 0 \qquad \frac{15}{(15+15)} = 0.5 \qquad \frac{30}{(0+30)} = 1$$

$$*)\ AMF:\frac{methyl}{(umethyl+methyl)} \qquad \bigcirc umethyl \qquad \bullet methyl$$

Fig.3

EP 4 282 984 A1

**(a)**

Learning methylation pattern configuration of continued 3 CpG sites within each WGBS read

WGBS read

Patterns (8 configurations possible)

...

Summarizing configuration frequency per each sample per segment (using all reads)

$$\text{proportion of each configuration}(x) = \frac{N_x}{N_{all}}$$

**(b)**

## healthy samples

| donor | type | Segment1 | segment2 |
|---|---|---|---|
| Sample1 | M | 0.88 | 0.00 |
| | U | 0.12 | 0.99 |
| Sample2 | M | 0.70 | 0.01 |
| | U | 0.25 | 0.97 |
| Sample | M | 0.65 | 0.17 |
| | U | 0.15 | 0.82 |
| ... | M | ... | ... |
| | U | ... | ... |
| summary (median) | M | 0.78 | 0.01 |
| | U | 0.20 | 0.98 |
| threshold | | 0.6 | |
| Major pattern | | methyl | unmethyl |

**(c)**

## healthy samples

| donor | Segment1 | Segment2 |
|---|---|---|
| Abnormal configuration | unmethyl | methyl |
| Sample1 | 0.12 | 0.00 |
| Sample2 | 0.25 | 0.01 |
| Sample3 | 0.15 | 0.17 |
| ... | ... | ... |

## Cancer samples

| donor | Segment1 | Segment2 |
|---|---|---|
| Abnormal configuration | unmethyl | methyl |
| Sample1 | 0.89 | 0.32 |
| Sample2 | 0.77 | 0.44 |
| Sample3 | 0.56 | 0.26 |
| ... | ... | ... |

Fig.4

$$CNR = \log_2 \frac{3}{2} = 0.585$$

3 copy

Cancer sample   2 copy

1 copy

**Healthy sample**                                                                 2 copy

$$CNR = \log_2 \frac{2}{2} = 0$$                    $$CNR = \log_2 \frac{1}{2} = -1$$

Fig.5

**Fragment size distribution**

**Fragment Size Distribution
Healthy cfDNA (n=47)**

**Fragment Size Distribution
Colon cfDNA (n=81)**

Fig.6

Fig.7

(a)

| | | CRC/HCC/BC | Healthy |
|---|---|---|---|
| | AMF | | |
| Cancer | | 102 | 3 |
| Non-cancer | | 3 | 39 |
| | | Sensitivity (97.1%) | Specificity (92.9%) |

(b)

| | | CRC/HCC/BC | Healthy |
|---|---|---|---|
| | AMPF | | |
| Cancer | | 100 | 2 |
| Non-cancer | | 5 | 40 |
| | | Sensitivity (95.2%) | Specificity (95.2%) |

(c)

| | | CRC/HCC/BC | Healthy |
|---|---|---|---|
| | CNR | | |
| Cancer | | 102 | 4 |
| Non-cancer | | 3 | 38 |
| | | Specificity (97.1%) | Sensitivity (90.5%) |

(d)

| | | CRC/HCC/BC | Healthy |
|---|---|---|---|
| | FragRatio | | |
| Cancer | | 103 | 2 |
| Non-cancer | | 2 | 40 |
| | | Specificity (98.1%) | Sensitivity (95.2%) |

Fig.8

Fig.9

**(a)**

| AMF | BC | CRC | HCC | Precision |
|---|---|---|---|---|
| BC | 23 | 1 | 0 | 95.8% |
| CRC | 5 | 50 | 2 | 87.7% |
| HCC | 0 | 2 | 22 | 91.7% |
| Recall | 82.1% | 94.3% | 91.7% | 90.5% |

**(b)**

| AMPF | BC | CRC | HCC | Precision |
|---|---|---|---|---|
| BC | 23 | 6 | 0 | 79.3% |
| CRC | 5 | 45 | 3 | 84.9% |
| HCC | 0 | 2 | 21 | 91.3% |
| Recall | 82.1% | 84.9% | 87.5% | 84.8% |

**(c)**

| CNR | BC | CRC | HCC | Precision |
|---|---|---|---|---|
| BC | 26 | 0 | 0 | 100% |
| CRC | 2 | 53 | 4 | 89.8% |
| HCC | 0 | 0 | 20 | 100% |
| Recall | 92.8% | 100% | 83.3% | 94.3% |

**(d)**

| Frag Ratio | BC | CRC | HCC | Precision |
|---|---|---|---|---|
| BC | 21 | 11 | 4 | 58.3% |
| CRC | 7 | 42 | 3 | 80.8% |
| HCC | 0 | 0 | 17 | 100% |
| Recall | 75.0% | 79.2% | 70.8% | 76.2% |

Fig.10

Probabilities

| Ensemble | BC | CRC | HCC | Precision |
|----------|------|------|------|-----------|
| BC | 27 | 1 | 0 | 96.4% |
| CRC | 1 | 52 | 3 | 92.8% |
| HCC | 0 | 0 | 21 | 100% |
| Recall | 96.4% | 98.1% | 87.5% | 95.2% |

## INTERNATIONAL SEARCH REPORT

| International application No. |
|---|
| **PCT/KR2022/012252** |

**A. CLASSIFICATION OF SUBJECT MATTER**

**C12Q 1/6886**(2018.01)i; **G16B 40/00**(2019.01)i

According to International Patent Classification (IPC) or to both national classification and IPC

**B. FIELDS SEARCHED**

Minimum documentation searched (classification system followed by classification symbols)

C12Q 1/6886(2018.01); C12Q 1/6827(2018.01); G06F 19/22(2011.01); G16H 50/30(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 메틸화 패턴(methylation pattern), 복제수 비율(copy number ratio), 절편 크기 비율(fragment size ratio), 시퀀싱(sequencing), cfDNA, CpG, 암(cancer)

**C. DOCUMENTS CONSIDERED TO BE RELEVANT**

| Category* | Citation of document, with indication, where appropriate, of the relevant passages | Relevant to claim No. |
|---|---|---|
| Y | US 2019-0316209 A1 (GRAIL, INC.) 17 October 2019 (2019-10-17)<br>See claims 1-27; paragraphs [0154]-[0155], [0169]-[0171] and [0498]; and figures 1A-1D. | 1,3-9 |
| A | | 2 |
| Y | MA, X. et al. Multi-dimensional fragmentomic assay for ultrasensitive early detection of colorectal advanced adenoma and adenocarcinoma. Journal of Hematology & Oncology. electronic publication 26 October 2021, vol. 14, thesis no. 175, pp. 1-4.<br>See abstract; pages 1-3; and figures 1-2. | 1,3-9 |
| Y | ZHANG, X. et al. Ultrasensitive and affordable assay for early detection of primary liver cancer using plasma cell-free DNA fragmentomics. Hepatology. 25 December 2021, vol. 76, pp. 317-329.<br>See abstract; pages 318-319, 322 and 327; and figures 1-2. | 1,3-9 |
| A | KR 10-2020-0032127 A (UNIVERSITY HEALTH NETWORK et al.) 25 March 2020 (2020-03-25)<br>See abstract; and claims 1-10 and 27-38. | 1-9 |

☑ Further documents are listed in the continuation of Box C.    ☑ See patent family annex.

| | |
|---|---|
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>**26 December 2022** | Date of mailing of the international search report<br>**27 December 2022** |
| Name and mailing address of the ISA/KR<br>**Korean Intellectual Property Office<br>Government Complex-Daejeon Building 4, 189 Cheongsa-ro, Seo-gu, Daejeon 35208**<br>Facsimile No. **+82-42-481-8578** | Authorized officer<br><br><br>Telephone No. |

Form PCT/ISA/210 (second sheet) (July 2022)

Form PCT/ISA/210 (second sheet) (July 2022)

**INTERNATIONAL SEARCH REPORT**

| International application No. |
|---|
| **PCT/KR2022/012252** |

**C.    DOCUMENTS CONSIDERED TO BE RELEVANT**

| Category* | Citation of document, with indication, where appropriate, of the relevant passages | Relevant to claim No. |
|---|---|---|
| A | KR 10-2017-0125044 A (THE CHINESE UNIVERSITY OF HONG KONG) 13 November 2017 (2017-11-13)<br>        See entire document. | 1-9 |

**INTERNATIONAL SEARCH REPORT**
Information on patent family members

International application No.

**PCT/KR2022/012252**

| Patent document cited in search report | | | Publication date (day/month/year) | Patent family member(s) | | | Publication date (day/month/year) |
|---|---|---|---|---|---|---|---|
| US | 2019-0316209 | A1 | 17 October 2019 | AU | 2019-253112 | A1 | 29 October 2020 |
| | | | | CA | 3096678 | A1 | 17 October 2019 |
| | | | | CN | 112204666 | A | 08 January 2021 |
| | | | | EP | 3776555 | A2 | 17 February 2021 |
| | | | | WO | 2019-200404 | A2 | 17 October 2019 |
| | | | | WO | 2019-200404 | A3 | 16 July 2020 |
| KR | 10-2020-0032127 | A | 25 March 2020 | BR | 112020000681 | A2 | 14 July 2020 |
| | | | | CA | 3069754 | A1 | 17 January 2019 |
| | | | | CA | 3080215 | A1 | 09 May 2019 |
| | | | | CN | 111094590 | A | 01 May 2020 |
| | | | | EP | 3652741 | A1 | 20 May 2020 |
| | | | | EP | 3704267 | A1 | 09 September 2020 |
| | | | | JP | 2020-537487 | A | 24 December 2020 |
| | | | | US | 2020-0308651 | A1 | 01 October 2020 |
| | | | | US | 2021-0156863 | A1 | 27 May 2021 |
| | | | | WO | 2019-010564 | A1 | 17 January 2019 |
| | | | | WO | 2019-084659 | A1 | 09 May 2019 |
| KR | 10-2017-0125044 | A | 13 November 2017 | AU | 2016-218631 | A1 | 17 August 2017 |
| | | | | AU | 2016-218631 | B2 | 10 March 2022 |
| | | | | AU | 2016-295616 | A1 | 15 February 2018 |
| | | | | AU | 2016-295616 | B2 | 02 June 2022 |
| | | | | CA | 2976303 | A1 | 18 August 2016 |
| | | | | CA | 2993362 | A1 | 26 January 2017 |
| | | | | CN | 107771221 | A | 06 March 2018 |
| | | | | CN | 107771221 | B | 02 November 2021 |
| | | | | CN | 108026572 | A | 11 May 2018 |
| | | | | CN | 113957124 | A | 21 January 2022 |
| | | | | DK | 3256605 | T3 | 14 March 2022 |
| | | | | DK | 3325664 | T3 | 07 March 2022 |
| | | | | EP | 3256605 | A1 | 20 December 2017 |
| | | | | EP | 3256605 | B1 | 09 February 2022 |
| | | | | EP | 3325664 | A1 | 30 May 2018 |
| | | | | EP | 3325664 | B1 | 29 December 2021 |
| | | | | EP | 3967775 | A1 | 16 March 2022 |
| | | | | EP | 4012715 | A1 | 15 June 2022 |
| | | | | HK | 1244515 | A1 | 10 August 2018 |
| | | | | HK | 1247645 | A1 | 28 September 2018 |
| | | | | HK | 1251018 | A1 | 18 January 2019 |
| | | | | HK | 1251264 | A1 | 25 January 2019 |
| | | | | IL | 257055 | A | 29 March 2018 |
| | | | | IL | 257055 | B | 01 January 2022 |
| | | | | IL | 288622 | A | 01 February 2022 |
| | | | | JP | 2018-512048 | A | 10 May 2018 |
| | | | | JP | 2018-524991 | A | 06 September 2018 |
| | | | | JP | 2021-061861 | A | 22 April 2021 |
| | | | | JP | 2021-184732 | A | 09 December 2021 |
| | | | | JP | 6829211 | B2 | 10 February 2021 |
| | | | | JP | 6931236 | B2 | 01 September 2021 |
| | | | | JP | 7168247 | B2 | 09 November 2022 |
| | | | | KR | 10-2018-0031742 | A | 28 March 2018 |

Form PCT/ISA/210 (patent family annex) (July 2022)

**INTERNATIONAL SEARCH REPORT**
Information on patent family members

| International application No. |
| --- |
| **PCT/KR2022/012252** |

| Patent document cited in search report | Publication date (day/month/year) | Patent family member(s) | | | Publication date (day/month/year) |
| --- | --- | --- | --- | --- | --- |
| | | PT | 3325664 | T | 03 March 2022 |
| | | SG | 10202106935 | A | 30 August 2021 |
| | | SG | 11201706529 | A | 28 September 2017 |
| | | TW | 201700732 | A | 01 January 2017 |
| | | TW | 201718872 | A | 01 June 2017 |
| | | TW | 202142697 | A | 16 November 2021 |
| | | TW | 202146657 | A | 16 December 2021 |
| | | TW | I730973 | B | 21 June 2021 |
| | | TW | I740817 | B | 01 October 2021 |
| | | US | 10240209 | B2 | 26 March 2019 |
| | | US | 10453556 | B2 | 22 October 2019 |
| | | US | 11168370 | B2 | 09 November 2021 |
| | | US | 2017-0024513 | A1 | 26 January 2017 |
| | | US | 2017-0073774 | A1 | 16 March 2017 |
| | | US | 2019-0153541 | A1 | 23 May 2019 |
| | | US | 2020-0005895 | A1 | 02 January 2020 |
| | | US | 2020-0005896 | A1 | 02 January 2020 |
| | | US | 2020-0005897 | A1 | 02 January 2020 |
| | | WO | 2016-127944 | A1 | 18 August 2016 |
| | | WO | 2017-012592 | A1 | 26 January 2017 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **METZKER, M.** *Nature Biotechnology Reviews,* 2010, vol. 11, 31-46